# EUROPÄISCHE PATENTSCHRIFT

(11) **EP 1 179 324 B1**
(45) Veröffentlichungstag und Bekanntmachung des Hinweises auf die Patenterteilung: **30.04.2008**
(21) Anmeldenummer: 01118344.9
(22) Anmeldetag: 27.07.2001
(51) Int. Cl.: A61F 2/34

(54) **Modularer Pfannenersatz**
Modular cup replacement
Remplacement modulaire pour une cupule

(30) Priorität: 29.07.2000 DE 10036987
(43) Veröffentlichungstag der Anmeldung: 13.02.2002
(73) Patentinhaber: Thurgauer Kantonalbank, 8570 Weinfelden (CH)
(72) Erfinder: Draenert, Klaus, 81545 München (DE)
(74) Vertreter: Rentsch & Partner

(56) Entgegenhaltungen:
- EP-A- 0 773 007
- DE-A- 3 200 340
- DE-A- 3 325 448
- DE-A- 4 425 235
- DE-U- 9 418 900
- US-A- 4 919 674

## Beschreibung

### Hintergrund der Erfindung

Der Erfolg der dauerhaften Verankerung von Hüftgelenkskomponenten verbindet sich mit den Publikationen von Sir John Charnley, der die Komponenten mit Knochenzement einzementiert hatte, beispielsweise publiziert im Journal of Bone and Joint Surgery : Charnley J(1960) Anchorage of the Femoral Head Prosthesis to the Shaft of the Femur JBJSB 42: 28-30. Auch die Kombination der zementierten Kunststoffpfanne geht auf J Charnley zurück (Low Friction Arthroplasty of the Hip: Theory and Practice - Heidelberg New York Berlin:Springer. Die Kombination mit Kunststoff führte jedoch zu unerwartet hohem Abrieb.

Daher war man bestrebt Kombinationen für die Artikulation zu finden, die geringen Abrieb erwarten liessen. Aus der Industrie war bekannt, dass die glattesten Oberflächen mit Keramiken erreicht wurden. Die Artikulation Keramik gegen Keramik lässt heute im Vergleich Polyäthylen-Metallkopf einen bis zu 200 mal geringeren Abrieb erwarten, beinhaltet jedoch das Problem, dass die Keramik nicht auf Biegung oder Scherung beansprucht werden sollte.

Die Kombination von Keramik und Keramik führte zu Metallimplantaten, die der Keramik Schutz bieten vor Biege- und Scherbeanspruchung, und die sich press-fit im Knochen verankern liessen. Dieses Flächenpressfit, welches auch mit Diamanten geschliffen werden kann (PA D 199 60 707.9) verlagert jedoch das Rotationszentrum (RZ) der Pfanne in die Tiefe des Acetabulums, was zu einer Medialisierung, einer Einwärtsverlagerung des Beines führt. Um dies zu verhindern, war man bestrebt, das RZ zu rekonstruieren, was mit der vorliegenden Erfindung gelungen ist.

### Stand der Forschung

Es gibt verschiedene Möglichkeiten die Pfanne zu verankern. Eine der gebräuchlichsten war die Zementierung einer UHMWPE (Ultra-High-Molecular-Weight-Poly-Ethylene) Pfanne mit Knochenzement, wie dies oben angeführt worden war. Man war dabei davon ausgegangen, dass die Zementierung der Pfanne denselben Gesetzen und damit Erfolgen folgte wie die Zementierung der Femurkomponente. Hierbei wurde man jedoch enttäuscht. Die Zementierung der Pfanne findet unter ganz anderen Bedingungen statt. Die Kunststoffpfanne wird auf einen mehr oder weniger kompakten Knochen aufzementiert, das heisst, dass der Knochenzement gar nicht in den Knochen eindringen kann, wenn keine Verankerungslöcher gemacht werden. Diese stellen jedoch Sanduhrverbindungen dar, die nur eine ungenügende Verankerung schaffen, da die Spongiosastruktur im Acetabulum zunächst enge Waben und nach proximal (gegen die Wirbelsäulen / Beckenverbindung zu) weiter werdende Waben erkennen lässt. Die Auffüllung erfolgt sozusagen gegen die morphologische Struktur, was dazu führt, dass sowohl die Aussteifung der Knochenstruktur als auch die Verankerung, das sogenannte Interlocking, ungenügend ist.

Nun gelingt es zwar, das kompakte Dach der Pfanne aufzuschleifen ohne die Stabilität der Leichtbaukonstruktion (Sandwichbauweise) zu zerstören, jedoch muss man hierfür ein Diamantinstrument einsetzen und den Knochenzement einsaugen. Da diese Technik erst neuerlich zur Verfügung steht (DPA 199 60 707.9) und das Abriebproblem der Kunststoffpfannen hinzukommt, war man in der jüngsten Vergangenheit bemüht, andere Verankerungsmöglichkeiten zu erproben.

Eines der gebräuchlichsten wurde das sogenannte Schraubprinzip, wo die künstliche Pfanne ohne Bindemittel direkt in den Knochen eingeschnitten und geschraubt wird. Die Schraubgewinde führen jedoch zur Knochennekrose und zu Beckenfrakturen.

Es wurde daraufhin versucht durch Vergrösserung der Verankerungsoberfläche dem Knochen die Möglichkeit zum Einwachsen zu geben (Porous Ingrowth Prinzip), doch auch hierbei kam es zu unvergleichlich hohen Lockerungsraten mit bindegewebiger Abkapselung der Implantate. Die Kombination mit der Verschraubung konnte das Problem auch nicht lösen.

Erst das Studium der Morphologie und Funktion der Facies lunata, das ist die sichelmondförmige Gelenkfläche der Pfanne, führte zu einem physiologischen Verankerungsprinzip: das Prinzip der Vorspannung im Knochen. Dies war nicht neu, am Beispiel der Pfanne jedoch leicht umzusetzen, da die Deformation der Gelenkfläche nahezu konzentrisch ist.

Es wurde daher möglich press-fit eine nahezu sphärische Komponente im Acetabulum, so wird die Pfanne im Beckenknochen bezeichnet, zu verankern. Die Feinheiten einer solchen Pfanne sind in der DPA 100 03 543.4 beschrieben.

Das Flächenpressfit erfordert die grossflächige Verblockung der Pfanne hinter den Äquator einer virtuellen Pfannenkugel; hierbei kommt es immer zu einer Verlagerung des RZs in die Tiefe der Pfanne. Dies wurde wenig beachtet, kann auch für die knöcherne Verankerung ein Vorteil sein, da die Hebelarme für eine Kippbewegung kürzer sind, d.h. das sogenannte Pfannenoffset ist kürzer, bedeutet aber in jedem Fall, dass das Bein als Ganzes weiter medialisiert wird, was zu einer pelvitrochanteren Insuffizienz führen kann. Dies bedeutet, dass die Muskeln relativ zu lang werden und einen ungünstigen Hebelarm haben. Aus diesem Grunde suchte man nach Lösungen, um den festen Sitz zu kombinieren mit einer Rekonstruktion der Lage des RZs . In der EP 0 694 294 B1 ist ein Einsatz beschrieben, der auf den ersten Blick geeignet erscheint, diesen Zweck zu erfüllen, dies jedoch nicht kann; die Zielsetzung dieser Erfindung ist das Lösen der Gelenkpfanne aus dem Mantelkonus, wobei ein Rand als Widerlager ausgebildet wird, der das Herausstemmen ermöglicht. Ein solcher Einsatz ist für die nachfolgend beschriebene Erfindung nicht einsetzbar, da eine Auflage am Schalenrand einen Presssitz verhindert. Dies konnte jedoch einfach und elegant mit der nachfolgenden Entwicklung gelöst werden.

Aus der EP 0 773 007 A1 ist ein Hüftgelenkspfannensystem gemäß den Merkalen des Oberbegriffs des Anspruchs 1 bekannt.

### Beschreibung der Erfindung

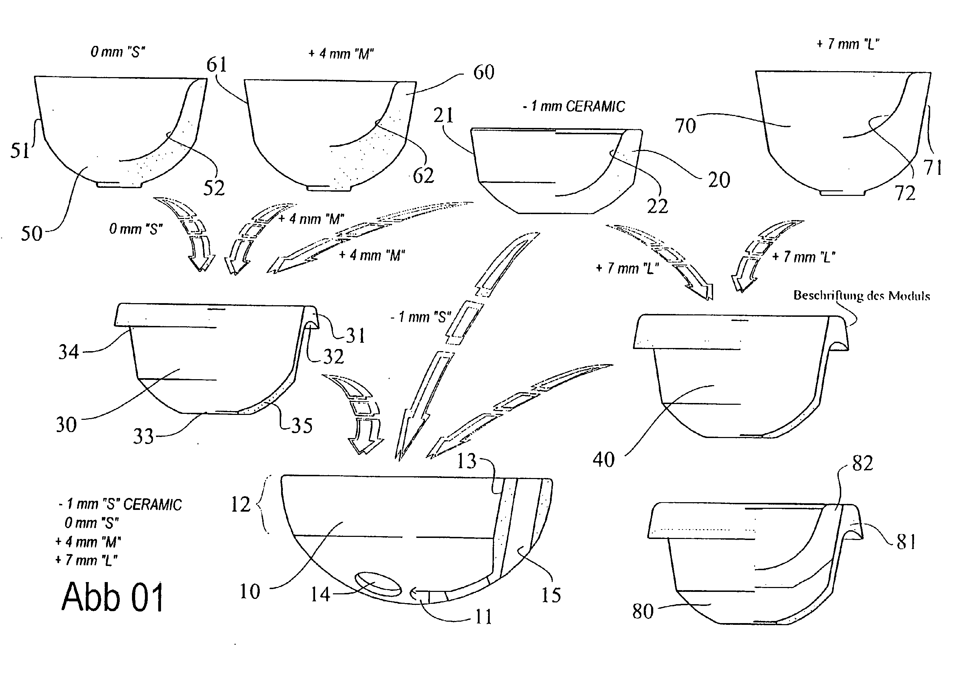

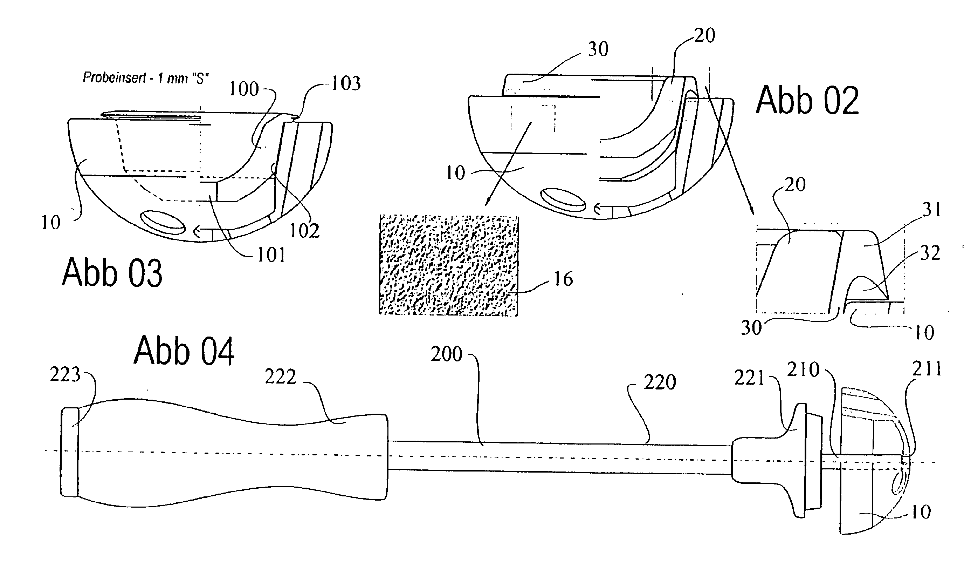

Die Erfindung besteht aus einem modularen Implantat, welches eine äussere Schale aufweist, wie dies in *(Abb 01*/*10)* dargestellt ist; diese ist im Dom abgeflacht *(Abb 01*/*11)* und weist ein sphärisches Band als Verankerungszone im Knochen auf *(Abb 01*/*12).* Die Innenkontour enspricht einem präzisen Konus mit definiertem Winkel *(**Abb. 01*/*13)*. Die Oberfläche dieser in diesem Beispiel dargestellten Titanpfanne weist eine Rauhigkeit von 80-100 Mikron auf *(**Abb. 02*/*16*), die Konusoberfläche eine definierte Rillung (Undulation) für die Aufnahme der Keramik *(**Abb 01*/*20),* bzw. des Insertmoduls *(Abb 01*/*30)* und *(Abb. 01*/*40).*

Die in diesem Falle aus Titan gefertigte Schale *(**Abb.01*/*10)* weist im Dom Perforationen auf, die die Masse reduzieren und die Gesamtelastizität der Konstruktion erhöhen *(Abb 01*/*14).* Die Bohrungen in der Wand des Titanimplantates (Abb 01/15) weisen dämpfende Eigenschaften gegenüber dem Knochen aus, wie in Finite Elementberechnungen gezeigt werden kann.

Das in die Titanschale *(**Abb.01*/*10)* einsetzbare Modul aus demselben Werkstoff *(**Abb. 01*/*30)* weist eine Ringauflage *(**Abb.01*/*31)* auf, die so gestaltet ist *(**Abb.01*/*32)*, dass es zu keiner Stresskonzentration an der Aufnahme der sphärischen Schale kommt. Der Boden *(**Abb.01*/*33)* des Moduls ist offen, der Konus *(**Abb.01*/*34)* weist eine für die Aufnahme des Keramikinlets spezifisch gerillte Oberfläche auf und ist hinterschnitten *(**Abb.01*/*35)*, damit die Subsidence gewährleistet ist, womit das Sichsetzen des Implantates umschrieben wird. Modul und artikulierenden Insert können im Hinblick auf die Langzeitstabilität fest mit einander verbunden sein. *(**Abb.01*/*80*).

Das Modul *(**Abb.01*/*30)* weist dabei eine relative Veränderung des Offset von 0 mm gegenüber dem reinen Keramikinlet *(**Abb.01*/ *20)* von - 1 mm, das Modul *(**Abb.01*/*60)* gar ein Offset von + 4 mm. Als Offset bezeichnet man bei der Pfanne den Abstand der Medianebene zum Rotationszentrum, wobei definitionsgemäss das physiologische RZ in seiner Topographie mit 0 mm definiert wird. Auch ein Modul mit + 7 mm Offset ist konstruiert, in *Abb. 01*/*40* dargestellt. Das Modul kann einteilig sein und jeweils fest mit der artikulierenden Komponente aus Keramik, HDPE, Metall oder einem anderen abriebfesten Material verbunden sein, was von Vorteil ist.

Diese Verlagerung des Offset kann bei den Titan /HDPE Varianten leichter in der Konstruktion des HDPE Inserts realisiert werden, wie dies in *Abb 01*/*50, Abb 01*/*60* und *Abb.01*/*70* dargestellt ist. Die konische Aussenseite des Insert *(Abb. 01*/ *61)* weist dabei eine spezifische Rillung für die Aufnahme im Titaninsert auf, welches das Kunststoffinsert formschklüssig aufnimmt *Abb.01*/*30 und* *Abb.01*/*40.* Die Innenflächen der Kunststoffinserts sind speziell geglättet; bei der einteiligen Ausführung fest mit dem Metall verbunden.

In derselben Weise kann ein artikulierendes Insert konstruiert werden, sowohl aus Kunststoff als auch aus Keramik, welches einen lateralen Wulst erkennen lässt, der der Luxation des künstlichen Kopfes entgegenwirkt; das Keramikinsert dieser Ausführung muss dabei von einem gesondert konstruierten Titanrücken vollständig unterstützt sein; beide Konstruktionen sind nicht extra dargestellt .

### Beispiel

Ein Patient mit fortgeschrittener Koxarthrose wird wie üblich auf dem Operationstisch gelagert und nach steriler Abdeckung und Vorbereitung des Operationsgebietes wird das Hüftgelenk beispielsweise über einen seitlichen Zugang freigelegt, die Kapsel eröffnet und das Femur luxiert und der Femurkopf hoch abgesetzt. Im Anschluss daran wird die Hüftpfanne eingestellt und mit einer scharfen Ruffle-Fräse der Knorpel entfernt

Sowie der Knochen frei liegt, wird der Rufflekorb gegen eine Diamantschleife gewechselt und mit der Grösse des Kopfes begonnen, das Bett sphärisch aufzuschleifen. Die nächst grössere Diamantschleife fräst sich auf den Pfannenboden, bis der Rand der sphärischen Hohlschleife in der Pfanne verschwindet. Es wird nun auf dem Einschlaginstrument *(Abb 04)* eine Titanschale aufgeschraubt und in dem geschliffenen Pfannenbett positioniert und mit Anteversion von 8° und einem Neigungswinkel der Eingangsebene von 38° tief in das knöcherne Bett eingeschlagen. Die Pfannenschale erreicht in allen Fällen einen absolut sicheren Pressitz. Das Einschlaginstrument wird herausgeschraubt und ein Probeinsert *(Abb 03*/*100)* eingesetzt. Das Probeinsert ist aus Kunststoff und ist in allen Varianten der Module verfügbar und beispielsweise durch verschiedene Farben eindeutig gekennzeichnet.

Der Boden des Probeinserts ist durchbrochen *(Abb.03*/*101),* die Aufnahme konisch *(Abb. 03*/*102)* korrespondierend zur Pfannenschale und die Auflage um die Zirkumferenz der Schale grosswulstig *(Abb.03*/ *103).* Das Probeinsert kann leicht wieder entfernt werden .

In der Folge wird das Femur präpariert und eine Komponente eingesetzt , danach wird ein Probekopf aufgesetzt und reponiert. Die Luxationstendenz und die Einstellung des RZ der Pfanne und des Femurs werden sorgfältig geprüft, gegebenenfalls wird eine Bildwandlerkontrolle durchgeführt. Nach erfolgter Prüfung wird das Probeinsert gegen ein Titanmodul mit Keramik, dieses kann zweiteilig oder besser einteilig sein, getauscht und ein definitiver Keramikkopf aufgesetzt.

Mit Einlegen der Drainagen, Muskel- und Fasziennähten, sowie Wundverschluss wird die Operation beendet.

### Modularer Pfannenersatz

| **Abb. 01** | |
|---|---|
| *Abb. 01*/*10* | Metallschale (Titan) zur Verankerung im Beckenknochen mit einer Abflachung und |
| *Abb. 01*/*11* | Durchbrechungen am Pfannenboden und einer |
| *Abb. 01*/*12* | bandförmigen, sphärischen Flächen-press fit-Verankerungszone mit |
| *Abb. 01*/*13* | einer präzisen Konusaufnahme für das Insert und |
| *Abb. 01*/*14* | einer bis mehrerer Durchbrüche am Pfannenboden und |
| *Abb. 01*/*15* | zirkulär um den Äquator angeordnete Bohrungen durch den Mantel. |
| | |
| *Abb. 01*/*20* | Keramikinsert mit Passung in *(Abb. 01*/*13)* durch den |
| *Abb. 01*/*21* | präzisionsgeschliffenen Konus und der |
| *Abb. 01*/*22* | präzisionsgeschliffenen, sphärischen Artikulation höchster Oberflächenglattheit. |
| *Abb. 01*/*30* | Metallmodul (Titan) für die Schale *(Abb. 01*/*10)* mit |
| *Abb. 01*/*31* | Auflage auf dem Schaleneingang und |
| *Abb. 01*/*32* | Struktur zur Vermeidung einer Sollbruchstelle und |
| *Abb. 01*/*33* | Durchbruch am Boden, sowie |
| *Abb. 01*/*34* | Präzisionskonusaufnahme für Insert *(Abb. 01*/*20)* oder andere mit Offset bis zu 4 mm und |
| *Abb. 01*/*35* | abgerundetem Pfannenboden. |
| | |
| *Abb. 01*/*40* | Metallmodul für HDPE- und Keramikinserts mit einem Offset bis 7 mm. |
| | |
| *Abb. 01*/*50* | HDPE-Insert in das Modul *(Abb. 01*/*30)* mit |
| *Abb. 01*/*51* | Präzisionskonus und |
| *Abb. 01*/*52* | Präzisionsartikulationsfläche höchster Glattheit für die Aufnahme einer Kugel mit 0 mm Offset des Inserts. |
| *Abb. 01*/*60* | HDPE-Insert für das Modul *(Abb. 01*/*30)* mit den analogen Merkmalen zu *(Abb. 01*/*51)* und *(Abb. 01*/*52)* analog *(Abb. 01*/*61* und *(Abb. 01*/*62)* mit einem Offset von 4 mm. |
| | |
| *Abb. 01*/*70* | HDPE-Insert für das Modul *(Abb. 01*/*40)* mit den analogen Merkmalen *(Abb. 01*/*71)* und *(Abb. 01*/*72)* zu *(Abb. 01*/*51)* und *(Abb. 01*/*52)* mit einem Offset von 7 mm. |
| *Abb. 01*/*80* | Einteiliges Modul, bestehend aus einem fest eingepressten Keramikinlet *(Abb. 01*/*81)* und einem metal back *(Abb. 01*/*82).* |
| *Abb. 02* | Metallschale *(Abb. 02*/*10)* mit isogenem Modul *(Abb. 02*/*30)* für die Aufnahme z. B. eines Keramikinserts *(Abb. 02*/*20)* mit |
| *Abb. 02*/*16* | einer Oberflächenrauhigkeit von 50 bis 140 µm, vorzugsweise 80 bis 100 µm. |
| | |
| ***Abb. 03*** | Metallschale *(Abb. 03*/*10)* mit einem Probeinsert *(Abb. 03*/*100)* mit |
| *Abb. 03*/*101* | perforiertem Boden und |
| *Abb. 03*/*102* | Präzisionskonus zur Aufnahme in der Schale und |
| *Abb. 03*/*103* | Kragenauflage mit der Möglichkeit zum leichten Hinterfahren im Falle eines Herausnehmens. |
| ***Abb. 04*** | Einsetzinstrument *(Abb. 04*/*200)* mit |
| *Abb. 04*/*210* | zentralem Stab mit |
| *Abb. 04*/*211* | Aufnahme für die Pfannenschale und |
| *Abb. 04*/*220* | Griffkonstruktion mit |
| *Abb. 04*/*221* | Schalenaufsatz und |
| *Abb. 04*/*222* | Handgriff und |
| *Abb. 04*/*223* | Hammeraufschlag. |

## Patentansprüche

1. Hüftgelenkspfannensystem zum Ersatz der natürlichen Hüftpfanne, bestehend aus einer äusseren Schale (10), wenigstens einem Insertmodul (30) und wenigstens einem Inserteinsatz (20) zur Aufnahme eines artikulierenden Kugelkopfes, wobei
- die äussere Schale (10) eine Innenkonusfläche (13) zur Aufnahme des Insertmoduls (30) aufweist, und
- das Insertmodul (30) eine Aussenkonusfläche (34) zum Anlegen an die korrespondierende Innenkonusfläche (13) der Schale (10) aufweist,
**dadurch gekennzeichnet, dass**
- das Insertmodul (30) eine sich radial nach aussen erstreckende Ringauflage (31) aufweist, welche an ihrer der Schale (10) zugewandten Unterseite zur Vermeidung von Stresskonzentration einen Freiraum (32) bogenförmig umschliesst, und dass
- das Insertmodul (30) einen offenen, abgerundeten Boden (33, 35) aufweist.

2. Hüftgelenkspfannensystem nach Anspruch 1, so beschaffen, dass die Aussenschale sphärisch ist mit abgeflachtem Dom und die Aufnahme für das Insertmodul konisch gestaltet ist.

3. Hüftgelenkspfannensystem nach Anspruch 1 oder 2, so beschaffen, dass die Neigung des Konus, der Konuswinkel, zwichen 3° und 15°; vorzugsweise 7°, beliegt was beispielsweise einer Steigung von 39/35 auf 13mm entspricht.

4. Hüftgolenkspfannensystem nach einem der Ansprüche 1 bis 3, so beschaffen, dass die Aussenschale aus Titan, einer Titanlegierung, Tantal, einer Tantallegierung, einer CoCrMo-Legierung oder aus rostfreiem Stahl und das insertmodul aus demselben Werkstoff besteht, im wesentlichen konusförmig gestaltet ist und ein mit dem künstlichen Kopf artikulierendes Insert aufnimmt, welches aus Keramik oder Kunststoff ist oder fest mit diesem verbunden ist..

5. Hüftgelenkspfannensyxtern nach einem der Ansprüche 1 bis 4 so beschaffen, dass ein HDPE Insert in einem Metallmodul eingesetzt ist oder fest mit diesem verbunden ist, welches die Deformation des Kunststoffes weitgehend verhindert.

6. Hüftgelenkspfannensystem nach einem der Ansprüche 2 bis 5, so beschaffen, dass in die sphärische Schale ein Probeinsert eingesetzt werden kann, welches sich leicht entfernen lässt.

7. Hüftgelenkspfannensystem nach einem der Ansprüche 1 bis 6, so beschaffen , dass für jedes Insertmodul ein korrespondierendes ein- oder zweiteiliges Probeinsert existiert, mit welchem eine Probereposition vorgenommen werden kann und welches so konstruiert ist, dass es leicht entfernt werden kann.

## Claims

1. A hip joint socket system for the replacement of the natural hip socket, consisting of an outer shell (10), at least one insertion module (30) and at least one insertion insert (20) for receiving an articulating ball head, wherein
- the outer shell (10) has an inner cone surface (13) for receiving the insertion module (30), and
- the insertion module (30) has an outer cone surface (34) for bearing on the corresponding inner cone surface (13) of the shell (10),
**characterised in that**
- the insertion module (30) comprises a radially outwardly extending annular rest (31), which at its lower side facing the shell (10) encloses a free space (32) in an arc-like manner, for avoiding stress concentration, and that
- the insertion module comprises an open, rounded base (33, 35).

2. A hip joint socket system according to claim 1, designed such that the outer shell is spherical with a flattened dome, and the receiver for the insertion module is designed in a conical manner.

3. A hip joint socket system according to claim 1 or 2, designed such that the inclination of the cone, the cone angle, is between 3° and 15°, preferably 7°, which corresponds to a gradient of 39/35 to 13 mm.

4. A hip joint socket system according to one of the claims 1 to 3, designed such that the outer shell consists of titanium, a titanium alloy, tantalum, a tantalum alloy, a CoCrMo-alloy or of stainless steel, and the insertion module consists of the same material, is formed in an essentially cone-like manner and receives an insertion insert that articulates with the artificial head, and which is of ceramic or plastic, or is firmly connected to the insertion module.

5. A hip joint socket system according to one of the claims 1 to 4, designed such that an HDPE insert is inserted in a metal module or is firmly connected to it, which largely prevents the deformation of the plastic.

6. A hip joint socket system according to one of the claims 2 to 5, designed such that a sample insert may be inserted into the spherical shell, and may be easily removed.

7. A hip joint socket system according to one of the claims 1 to 6, designed such that a corresponding single-part or two-part sample insert exists for each insertion module, with which a sample reposition may be carried out and which is designed such that it may be easily removed.

## Revendications

1. Système de cavité cotyloïde servant à remplacer le cotyle naturel, constitué d'une coquille extérieure (10), d'au moins un module d'insert (30) et d'au moins un insert (20) servant au logement d'une tête sphérique articulée,
- la coquille extérieure (10) présentant une surface conique intérieure (13) servant au logement du module d'insert (30), et
- le module d'insert (30) présentant une surface conique extérieure (34) servant à l'application au niveau de la surface conique intérieure correspondante (13) de la coquille (10),
**caractérisé en ce que**
- le module d'insert (30) présente un support annulaire (31) s'étendant radialement vers l'extérieur, lequel entoure en forme d'arc un espace libre (32) au niveau de sa face inférieure tournée vers la coquille (10), destiné à éviter la concentration de contraintes, et **en ce que**
- le module d'insert (30) présente un fond (33, 35) ouvert, arrondi.

2. Système de cavité cotyloïde selon la revendication 1, constitué de manière telle que la coquille extérieure est sphérique avec un dôme aplati et le logement pour le module d'insert est réalisé de manière conique.

3. Système de cavité cotyloïde selon la revendication 1 ou 2, constitué de manière telle que l'inclinaison du cône, l'angle du cône, est comprise entre 3° et 15°, de préférence égale à 7°, ce qui correspond par exemple à une pente de 39/35 sur 13 mm.

4. Système de cavité cotyloïde selon l'une quelconque des revendications 1 à 3, constitué de manière telle que la coquille extérieure est en titane, en un alliage de titane, en tantale, en un alliage de tantale, en un alliage CoCrMo ou en acier inoxydable et le module d'insert est dans le même matériau, présente essentiellement une forme conique et reçoit un insert s'articulant avec la tête artificielle, lequel est en céramique ou en plastique ou est relié solidairement à celle-ci.

5. Système de cavité cotyloïde selon l'une quelconque des revendications 1 à 4, constitué de manière telle qu'un insert HDPE est inséré dans un module métallique ou est relié solidairement à celui-ci, lequel empêche largement la déformation du plastique.

6. Système de cavité cotyloïde selon l'une quelconque des revendications 2 à 5, constituée de manière telle qu'un insert d'essai, lequel peut facilement s'enlever, peut être inséré dans la coquille sphérique.

7. Système de cavité cotyloïde selon l'une quelconque des revendications 1 à 6, constitué de manière telle qu'il existe pour chaque module d'insert, un insert d'essai correspondant à un ou deux éléments, avec lequel il est possible de procéder à un repositionnement d'essai et lequel est construit de sorte à pouvoir être facilement enlevé.
